# EUROPÄISCHE PATENTSCHRIFT

(11) **EP 4 076 392 B1**
(45) Veröffentlichungstag und Bekanntmachung des Hinweises auf die Patenterteilung: **26.02.2025**
(21) Anmeldenummer: 20842699.9
(22) Anmeldetag: 18.12.2020
(51) Int. Cl.: A61K 9/00, A61K 9/70, A61K 47/14, A61K 9/26, A61K 47/26, A61K 47/32, A61K 31/135, A61K 31/165

(54) **LÖSLICHE RÜCKSCHICHT FÜR OTF**
SOLUBLE REAR LAYER FOR OTF
COUCHE ARRIÈRE SOLUBLE POUR OTF

(30) Priorität: 20.12.2019 DE 102019135432
(43) Veröffentlichungstag der Anmeldung: 26.10.2022
(73) Patentinhaber: LTS Lohmann Therapie-Systeme AG, 56626 Andernach (DE)
(72) Erfinder: MÜLLER, Markus, 53840 Troisdorf (DE)
(74) Vertreter: Meissner Bolte Partnerschaft mbB
(86) Internationale Anmeldenummer: PCT/EP2020/087195
(87) Internationale Veröffentlichungsnummer: WO 2021/123289

(56) Entgegenhaltungen:
- DE-A1- 102005 007 059
- MASEK JOSEF ET AL: "Multi-layered nanofibrous mucoadhesive films for buccal and sublingual administration of drug-delivery and vaccination nanoparticles - important step towards effective mucosal vaccines", JOURNAL OF CONTROLLED RELEASE, ELSEVIER, AMSTERDAM, NL, vol. 249, 25 July 2016 (2016-07-25), pages 183 - 195, XP029927819, ISSN: 0168-3659, DOI: 10.1016/J.JCONREL.2016.07.036
- SOLOMONIDOU D ET AL: "Effect of carbomer concentration and degree of neutralization on the mucoadhesive properties of polymer films", JOURNAL OF BIOMATERIALS SCIENCE. POLYMER EDITION, VSP, UTRECHT, NL, vol. 12, no. 11, 1 January 2001 (2001-01-01), pages 1191 - 1205, XP009028488, ISSN: 0920-5063, DOI: 10.1163/156856201753395743

## Beschreibung

Die vorliegende Erfindung betrifft einen mehrschichtigen oralen Dünnfilm, ein Verfahren zu dessen Herstellung, sowie die Verwendung eines solchen oralen Dünnfilms als Arzneimittel.

Orale Dünnfilme sind dünne, mindestens einen pharmazeutisch aktiven Wirkstoff enthaltende Filme, die direkt in den Mundraum gelegt oder an die Mundschleimhaut angelegt werden und sich dort auflösen bzw. aufquellen und dabei den Wirkstoff abgeben. Dabei handelt es sich insbesondere um dünne, ein oder mehrschichtige, wirkstoffhaltige Filme auf Polymerbasis, die, wenn sie auf eine Schleimhaut, insbesondere die Mundschleimhaut, aufgebracht werden, den Wirkstoff direkt in diese abgeben können. Die sehr gute Durchblutung der Mundschleimhaut sorgt für einen schnellen Übergang des Wirkstoffs in den Blutkreislauf. Dieses Darreichungssystem hat den Vorteil, dass der Wirkstoff größtenteils durch die Schleimhaut resorbiert wird und damit der "First-Pass Metabolismus", der bei der konventionellen Darreichungsform eines Wirkstoffs in Tablettenform auftritt, vermieden wird. Der Wirkstoff kann in dem Film gelöst, emulgiert oder dispergiert werden.

Aus dem Stand der Technik bekannte orale Dünnfilme haben den Nachteil, dass sie, wenn sie längere Zeit an einer Stelle auf der Schleimhaut eines Patienten verbleiben sollen, einer dauerhaften Erosion ausgesetzt sind. Dies führt dazu, dass ein großer Teil des Materials abgeschluckt wird und so nicht die gewünschte Dauer am Applikationsort verweilt. Die Verweildauer kann aber durchaus für den transmucosalen Transport des pharmazeutisch aktiven Wirkstoffs von entscheidender Bedeutung sein.

Durch eine Schutzschicht auf der Rückseite kann verhindert werden, dass Flüssigkeit in die Formulierung eindringt und diese zu schnell auflöst, so dass der Wirkstoff maximale Zeit an der Applikationsstelle verweilt um eine größtmögliche Permeation durch die Mucosa oder eine verzögerte Freisetzung zu erreichen. Ein weiterer Effekt der Rückschicht ist, dass sie verhindert, dass sich der verabreichte Film von dem Applikationsort löst und an anderer Stelle wie z.B. den Zähnen anhaftet.

Als Material für solche Rückschichten werden häufig unlösliche oder sehr langsam lösliche Polymere bzw. Polymerfolien eingesetzt. Diese haben jedoch den Nachteil, dass sie nach Beendigung der Applikation entnommen oder abgeschluckt werden musste.

Rückschichten aus langsam löslichen Polymeren haben zudem den Nachteil, dass sie auf langkettigen, hochmolekularen Polymeren basieren. Diese sind auf Grund ihrer hohen Viskosität schwer zu verarbeiten (lange Trocknungsdauern, unregelmäßige Filme). Des Weiteren neigen diese dazu, die Viskosität des Speichels im Mundraum zu erhöhen, wodurch ein schleimiges Gefühl im Mundraum entsteht.

Masek Josef et al. (Journal of controlled release, Elsevier, Amsterdam, NL; Bd. 249, 25. Juli 2016, Seiten 183-195) offenbart einen mehrschichtigen oralen Dünnfilm, der eine Matrixschicht und eine Rückschicht enthält, wobei die Rückschicht aus einem oral löslichen Polymer besteht, der die Matrixschicht vor Erosion schützt.

Die Aufgabe der vorliegenden Erfindung besteht darin, vorstehend genannte Nachteile des Standes der Technik zu beheben. Insbesondere besteht die Aufgabe der vorliegenden Erfindung darin, einen mehrschichtigen oralen Dünnfilm bereitzustellen, der mindestens eine Rückschicht aufweist, deren Auflösungszeit sich variieren und damit einstellen lässt, und die sich rückstandslos auflösen lässt ohne ein unangenehmes Mundgefühl zu erzeugen.

Obige Aufgabe wird durch einen mehrschichtigen oralen Dünnfilm nach Anspruch 1 gelöst, der eine mindestens ein Polymer und mindestens einen pharmazeutisch aktiven Wirkstoff enthaltende Matrixschicht und mindestens eine Rückschicht umfasst, wobei die mindestens eine Rückschicht eine Schichtdicke von 10 µm bis 500 µm aufweist und mindestens ein Polymer, das freie Carboxylgruppen aufweist, umfasst, wobei 10 bis 100% der freien Carboxylgruppen des mindestens einen Polymers, das freie Carboxylgruppen umfasst, neutralisiert als Salz vorliegen, und wobei das mindestens eine Polymer, das freie Carboxylgruppen umfasst, in einer Menge von 10 bis 99 Gew.-%, bezogen auf das Gesamtgewicht der Rückschicht, in der Rückschicht vorhanden ist.

Ein solcher mehrschichtiger oraler Dünnfilm hat den Vorteil, dass durch eine gezielte Einstellung des Neutralisationsgrades des mindestens einen Polymers, das freie Carboxylgruppen umfasst, eine Rückschicht bereitgestellt werden kann, die sich nach gewünschter Zeit rückstandsfrei gelöst hat. Dadurch, dass das Polymer im nicht neutralisierten Zustand vorzugsweise wasserunlöslich ist und im voll neutralisierten Zustand sehr gut wasserlöslich ist, kann die Auflösegeschwindigkeit beliebig von unlöslich bis schnell löslich eingestellt werden. Die Rückschicht schützt so durch langsame Löslichkeit den applizierten Film zuverlässig und über die gewünschte Dauer.

Auch durch Überstreichen mit der Zunge kann keine erhöhte Erosion erreicht werden, da die Auflösung nicht mechanisch beschleunigt werden kann, sondern abhängig von der Pufferkapazität des vorhandenen Speichels und des nachproduzierten Speichels ist.

Ferner ist es vorteilhaft, dass aufgrund der guten Löslichkeit im neutralisierten Zustand die Rückschicht kein unangenehmes Mundgefühl auslöst.

Zusätzlich kann die Rückschicht mit Farbstoff eingefärbt werden, um eine bessere Sichtbarkeit zu erlangen.

Weiter ist es möglich Geschmacksstoffe einzuarbeiten, um das Mundgefühl während der Anwendung zu verbessern, was einen großen Vorteil gegenüber Folien darstellt.

In der vorliegenden Beschreibung des oralen Dünnfilms kann "umfassend" auch "bestehend aus" bedeuten.

Unter Rückschicht wird eine Schicht des mehrschichtigen oralen Dünnfilms verstanden, die eine der äußersten Schichten des mehrschichtigen oralen Dünnfilms darstellt.

Das mindestens eine Polymer, das freie Carboxylgruppen umfasst, ist vorzugsweise ein in Wasser schwer lösliches, sehr schwer lösliches oder praktisch unlösliches Polymer gemäß der folgenden Tabelle.

Löslichkeit bei 15 °C bis 25 °C:

| Bezeichnung | V(Lösungsmittel) in ml je g Substanz | g·l⁻¹ Lösungsmittel |
|---|---|---|
| sehr leicht löslich | < 1 | >1000 |
| leicht löslich | 1 bis 10 | 100 bis 1000 |
| löslich | 10 bis 30 | 33 bis 100 |
| wenig löslich | 30 bis 100 | 10 bis 33 |
| schwer löslich | 100 bis 1000 | 1 bis 10 |
| sehr schwer löslich | 1000 bis 10000 | 0,1 bis 1 |
| praktisch (nahezu) unlöslich | > 10000 | < 0,1 |

Es hat sich gezeigt, dass die Löslichkeit von Polymeren, die freie Carboxylgruppen umfassen, in Wasser durch teilweise Neutralisierung bzw. der Zugabe von mindestens einer Base variiert werden kann. Übertragen auf die Rückschicht des mehrschichtigen oralen Dünnfilms bedeutet das, dass die Zerfallszeit der Rückschicht im Mund des Patienten variiert werden kann.

Der erfindungsgemäße mehrschichtige orale Dünnfilm ist dadurch gekennzeichnet, dass mindestens 10 %, besonders bevorzugt von 15 bis 100% der freien Carboxylgruppen des mindestens einen Polymers, das freie Carboxylgruppen umfasst, neutralisiert als Salz vorliegen.

Vorzugsweise liegen 15 bis 100% oder 20 bis 100% oder 25 bis 100% oder 30 bis 100% oder 35 bis 100% oder 40 bis 100% oder 45 bis 100% oder 50 bis 100% oder 60 bis 100% oder 70 bis 100% oder 80 bis 100% oder 90 bis 100% der freien Carboxylgruppen des mindestens einen Polymers, das freie Carboxylgruppen umfasst, neutralisiert als Salz vor.

Gemäß der Erfindung liegen 10 bis 100%, vorzugsweise 15 bis 100% oder 20 bis 95% oder 25 bis 90% oder 30 bis 85% oder 35 bis 80% oder 40 bis 75% oder 45 bis 70% oder 50 bis 65% oder 55 bis 60% der freien Carboxylgruppen des mindestens einen Polymers, das freie Carboxylgruppen umfasst, neutralisiert als Salz vor.

Sind, entgegen der Erfindung, 0% der freien Carboxylgruppen des mindestens einen Polymers, das freie Carboxylgruppen umfasst neutralisiert, so ist das Polymer vorzugsweise unlöslich. Sind etwa 10% der freien Carboxylgruppen des mindestens einen Polymers, das freie Carboxylgruppen umfasst neutralisiert, so weist das Polymer vorzugsweise einen pH von etwa 4 auf und ist damit sehr langsam löslich.

Sind etwa 15% bis 100% der freien Carboxylgruppen des mindestens einen Polymers, das freie Carboxylgruppen umfasst neutralisiert, so weist das Polymer vorzugsweise einen pH von etwa 4,6 bis 7 auf.

Der erfindungsgemäße mehrschichtige orale Dünnfilm ist ferner vorzugweise dadurch gekennzeichnet, dass die mindestens eine Rückschicht einen pH von 3,5 bis 7,5, vorzugsweise von 4 bis 7 besonders 4,5 bis 6,5 aufweist.

Der pH der Rückschicht entspricht dem pH der Lösung oder Suspension einer wässrigen Lösung aller Inhaltsstoffe der Rückschicht bei 20°C. Die wässrige Lösung kann dabei ausschließlich Wasser als Lösungsmittel, aber auch Mischungen von Wasser mit organischen Lösungsmitteln umfassen. Als organische Lösungsmittel eigenen sich prinzipiell alle mit Wasser mischbaren organischen Lösungsmittel. Bevorzugt sind Ketone, Alkohole und/oder Ester, insbesondere Aceton oder Ethanol.

Ist mehr als ein Polymer, das freie Carboxylgruppen umfasst in der Rückschicht vorhanden, so beziehen sich die genannten Werte für den Neutralisationsgrad der freien Carboxylgruppen auf die Summe aller vorhandenen Carboxylgruppen.

Der erfindungsgemäße mehrschichtige orale Dünnfilm ist ferner dadurch gekennzeichnet, dass das mindestens eine Polymer, das freie Carboxylgruppen umfasst, in einer Menge von 10 bis 99 Gew.-%, vorzugsweise 20 bis 95 Gew.-%, besonders bevorzugt 25 bis 90 Gew.-%, bezogen auf das Gesamtgewicht der Rückschicht, in der Rückschicht vorhanden ist.

Es können auch Mischungen mehrerer Polymere, die freie Carboxylgruppen umfassen, eingesetzt werden. Hierbei liegt die Summe aller Polymeren, die freie Carboxylgruppen umfassen, bei einer Menge von 10 bis 99 Gew.-%, vorzugsweise 20 bis 95 Gew.-%, besonders bevorzugt 25 bis 90 Gew.-%, bezogen auf das Gesamtgewicht der Rückschicht.

Der erfindungsgemäße mehrschichtige orale Dünnfilm ist ferner vorzugweise dadurch gekennzeichnet, dass der Gehalt an freien Carboxylgruppen in dem Polymer, das freie Carboxylgruppen umfasst, bezogen auf die mittlere Polymermasse 10 bis 40 Gew.-%, vorzugsweise 15 bis 35 Gew.-%, besonders bevorzugt 20 bis 30 Gew.-, beträgt.

Ist mehr als ein Polymer, das freie Carboxylgruppen umfasst in der Rückschicht vorhanden, so beziehen sich diese genannten Werte auf die Summe aller vorhandenen Carboxylgruppen.

Das mindestens eine Polymer, das freie Carboxylgruppen umfasst, umfasst vorzugsweise ein Polymer auf Basis von (Meth)Acrylsäure und/oder auf Basis eines Copolymers von (Meth)Acrylsäure und (Meth)Acrylsäureestern.

Ganz besonders bevorzugt umfasst das mindestens eine Polymer, das freie Carboxylgruppen umfasst, ein Copolymer von (Meth)Acrylsäure und (Meth)Acrylsäureestern, ganz besonders bevorzugt ein (Meth)Acrylsäure/Ethylacrylat-Copolymer.

Geeignete Polymere sind beispielsweise unter den Handelsnamen Kollicoat L100, Kollicoat L100-55, Kollicoat MAE Kollicoat Smart Seal (von BASF) oder Eudragit L, Eudragit S (Evonic) oder Acryl-EZE (Colorcon) erhältlich.

Diese Polymere haben den Vorteil, dass sie nahezu unlöslich in Wasser sind und deren Löslichkeit durch Zugabe von mindestens einer Base gut variiert werden kann. Teilweise sind einige dieser Polymere bereits von Herstellerseite vorneutralisiert.

Der erfindungsgemäße mehrschichtige orale Dünnfilm ist ferner vorzugsweise dadurch gekennzeichnet, dass die freien Carboxylgruppen des mindestens einen Polymers, das freie Carboxylgruppen umfasst, durch Zugabe mindestens einer Base neutralisiert wurden.

Die Art der Base ist nicht beschränkt, sondern es kann prinzipiell jede pharmazeutisch akzeptable Base verwendet werden. Besonders bevorzugt umfasst die Base ein Hydroxid, insbesondere ein Alkali- oder Erdalkalimetallhydroxid. Ganz besonders bevorzugt ist der Einsatz von NaOH.

Die zugegebene Menge der mindestens einen Base wird vorzugsweise so bemessen, dass das Äquivalenzverhältnis der Base zu den freien Carboxylgruppen des mindestens einen Polymers, das freie Carboxylgruppen umfasst, 1:10 bis 2:1, vorzugsweise 1:10 bis 1:1, beträgt.

Wird NaOH als Base zugegeben, so kann die zugegebene Menge vorzugsweise so bemessen werden, dass 0,5 bis 15 Gew.-% NaOH, bezogen auf das Gewicht des mindestens einen Polymers, das freie Carboxylgruppen umfasst, zugegeben werden.

Der erfindungsgemäße mehrschichtige orale Dünnfilm ist ferner vorzugsweise dadurch gekennzeichnet, dass die Rückschicht mindestens einen Weichmacher umfasst.

Geeignete Weichmacher umfassen dabei Zitronensäureester, Alpha Tocopherol, Benzyl Benzoat, Butylstearat, Chlorbutanol, Dibutylphalate, Dibutylsebacate, Diethylphalat, Dimethylphtalat, Dipropylenglycol, Glycerin, Glycerolmonostearat, Polyethylenglycol, Propylenglycol, Stearinsäure, Triacetin und/oder Tricaprylin, besonders bevorzugst Triethylcitrat.

Der erfindungsgemäße mehrschichtige orale Dünnfilm ist vorzugsweise dadurch gekennzeichnet, dass der mindestens ein Weichmacher in einer Menge von 1 bis 20 Gew.-%, vorzugsweise von 5 bis 15 Gew.-%, besonders bevorzugt von 8 bis 12 Gew.-%, bezogen auf das Gesamtgewicht der Rückschicht, in der Rückschicht des mehrschichtigen oralen Dünnfilm vorhanden ist.

Der erfindungsgemäße mehrschichtige orale Dünnfilm ist ferner vorzugsweise dadurch gekennzeichnet, dass die Matrixschicht mindestens ein wasserlösliches Polymer umfasst.

Wasserlösliche Polymere umfassen chemisch sehr unterschiedliche, natürliche oder synthetische Polymere, deren gemeinsames Merkmal ihre Löslichkeit in Wasser oder wässrigen Medien ist. Voraussetzung ist, dass diese Polymere eine für die Wasserlöslichkeit ausreichende Anzahl an hydrophilen Gruppen besitzen und nicht vernetzt sind. Die hydrophilen Gruppen können nicht-ionisch, anionisch, kationisch und/oder zwitterionisch sein.

Wasserlösliche Polymere weisen vorzugsweise eine Löslichkeit in Wasser gemäß der vorstehenden Tabelle von mindestens "löslich" auf.

Das mindestens eine wasserlösliche Polymer ist vorzugsweise ausgewählt aus der Gruppe bestehend aus Stärke und Stärkederivaten, Dextranen; Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose; Polyacrylsäuren, Polyacrylaten, Polyvinylpyrrolidonen, Vinvlpyrrolidon/Vinylacetat-Copolymer, Polyvinylalkoholen, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und natürlichen Gummen.

### Besonders bevorzugt umfasst das mindestens eine wasserlösliche Polymer Vinvlpyrrolidon/Vinylacetat-Copolymer

Der erfindungsgemäße mehrschichtige orale Dünnfilm ist vorzugsweise dadurch gekennzeichnet, dass das mindestens eine Polymer, vorzugsweise das wasserlösliche Polymer in einer Menge von 5 bis 95 Gew.-%, vorzugsweise von 10 bis 80 Gew.-%, bezogen auf das Gesamtgewicht der Matrixschicht, in der Matrixschicht des mehrschichtigen oralen Dünnfilm vorhanden ist.

Es sind auch Ausführungsformen des erfindungsgemäßen mehrschichtigen oralen Dünnfilms möglich, wobei das mindestens eine Polymer, vorzugsweise das wasserlösliche Polymer in einer Menge von 5 bis 50 Gew.-%, vorzugsweise von 10 bis 40 Gew.-%, bezogen auf das Gesamtgewicht der Matrixschicht, in der Matrixschicht des mehrschichtigen oralen Dünnfilm vorhanden ist.

Der mindestens eine pharmazeutisch aktive Wirkstoff unterliegt prinzipiell keiner Beschränkung, sondern ist vorzugsweise ausgewählt unter allen pharmazeutisch aktiven Wirkstoffen, die zur oralen und/oder transmucosalen Applikation geeignet sind.

Gemäß der vorliegenden Erfindung werden unter dem pharmazeutisch aktiven Wirkstoff auch alle pharmazeutisch akzeptablen Salze und Solvate des jeweiligen pharmazeutisch aktiven Wirkstoffs subsumiert.

Bevorzugt sind Wirkstoffe ausgewählt aus der Gruppe, bestehend aus den Wirkstoffklassen der Analgetika, Hormone, Hypnotika, Sedativa, Antiepileptika, Weckaminen, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormonen, Sexualhormonen, Antidiabetika, Antitumor-Wirkstoffen, Antibiotika, Chemotherapeutika und Narcotika, wobei diese Gruppe nicht abschließend ist.

Besonders bevorzugt handelt es sich bei dem mindestens einen pharmazeutisch aktiven Wirkstoff um Ketamin und/oder ein pharmazeutisch akzeptables Salz oder Solvat davon vor, bevorzugt Ketamin·HCl.

Unter Ketamin wird hierbei (S)-(±)-2-(2-Chlorphenyl)-2-(methylamino)cyclohexan-1-on, (R)-(±)-2-(2-Chlorphenyl)-2-(methylamino)cyclohexan-1-on, sowie das Racemat (RS)-(±)-2-(2-Chlorphenyl)-2-(methylamino)cyclohexan-1-on verstanden.

Besonders bevorzugt liegt jedoch (S)-Ketamin oder ein pharmazeutisch akzeptables Salz davon, insbesondere (S)-Ketamin·HCl, als einziges Stereoisomer von Ketamin vor, da die analgetische und anästhetische Potenz von (S)-Ketamin etwa dreifach höher als die der (R)-Form ist.

Der erfindungsgemäße mehrschichtige orale Dünnfilm ist ferner vorzugsweise dadurch gekennzeichnet, dass der mindestens eine pharmazeutisch aktive Wirkstoff, vorzugsweise das Ketamin, in einer Menge von 1 bis 50 Gew.-%, vorzugsweise von 10 bis 40 Gew.-%, besonders bevorzugt von 25 bis 35 Gew.-%, bezogen auf das Gesamtgewicht der Matrixschicht, in der Matrixschicht vorhanden ist.

Der erfindungsgemäße mehrschichtige orale Dünnfilm ist ferner vorzugsweise dadurch gekennzeichnet, dass die Matrixschicht jeweils ferner mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von etwa 0,1 bis 40 Gew.-%, vorzugsweise von 0,1 bis 30 Gew.-%, besonders bevorzugt von 0,1 bis 15 Gew.-%, bezogen auf das Gesamtgewicht der Matrixschicht, in dieser Schicht enthalten.

Der erfindungsgemäße mehrschichtige orale Dünnfilm ist ferner vorzugsweise dadurch gekennzeichnet, dass die Rückschicht ferner mindestens einen Hilfsstoff ausgewählt aus der Gruppe, umfassend Farbstoffe, Aromastoffe, Süßstoffe, geschmacksmaskierende Mittel, Emulgatoren, Enhancer, pH-Regulatoren, Feuchthaltemittel, Konservierungsmittel und/oder Antioxidationsmittel, umfasst.

Jeder dieser Hilfsstoffe ist vorzugsweise jeweils in einer Menge von etwa 0,1 bis 40 Gew.-%, vorzugsweise von 0,1 bis 30 Gew.-%, besonders bevorzugt von 0,1 bis 15 Gew.-%, bezogen auf das Gesamtgewicht der Rückschicht, in dieser Schicht enthalten.

Der erfindungsgemäße orale Dünnfilm besitzt vorzugsweise eine Fläche von etwa 0,5 cm² bis etwa 10 cm², besonders bevorzugt von etwa 2 cm² bis etwa 8 cm².

Der erfindungsgemäße orale Dünnfilm ist vorzugsweise dadurch gekennzeichnet, dass das Flächengewicht des oralen Dünnfilms etwa 10 bis 500 g/m², vorzugsweise etwa 100 bis 400 g/m², beträgt.

Das Flächengewicht der Rückschicht ist wichtig für die Kontrolle des Auflösungsverhaltens und die Funktion der Rückschicht, um den Wirkstoff vor dem Auflösen im Speichel zu schützen. Eine bestimmte Dicke ist erforderlich, um einen ausreichenden Schutz des Wirkstoffs sowie eine ausreichende Auflösungszeit zu gewährleisten, die normalerweise mindestens so lang sein sollte wie der aktive Permeationsbedarf und damit die Auflösungszeit der Matrixschicht.

Darüber hinaus ist es auch schwierig, insbesondere sehr dünne Schichten mit ausreichender Genauigkeit zu beschichten. Andererseits können dicke Schichten nicht nur ein unangenehmes Gefühl in der Mundhöhle hervorrufen, sondern auch schwierig herzustellen sein und dazu führen, dass sich die Schicht für die gewünschte (z.B. über Nacht) Anwendung zu lange auflöst. Im Ganzen wird bevorzugt, dass die Rückschicht ein Flächengewicht von mindestens 10 g/m², bevorzugter mindestens 20 g/m² oder am meisten bevorzugt mindestens 30 g/m² oder ein Flächengewicht von weniger als oder gleich 400 g/m², bevorzugter weniger als oder gleich 350 g/m² oder am meisten bevorzugt weniger als oder gleich 300 g/m² oder ein Flächengewicht von 10 bis 400 g/m², bevorzugter von 20 bis 350 g/m² oder am meisten bevorzugt von 30 bis 300 g/m² aufweist.

Dies entspricht erfindungsgemäss einer Schichtdicke von 10 µm bis 500 µm, besonders bevorzugt von etwa 20 µm bis etwa 300 µm.

Der erfindungsgemäße orale Dünnfilm ist vorzugsweise dadurch gekennzeichnet, dass sich die Trägerschicht in bestimmten Ausführungsformen in 5 Minuten oder mehr, vorzugsweise in 10 Minuten oder mehr, und bevorzugter in 15 Minuten oder mehr, oder in 12 Stunden oder weniger, vorzugsweise in 8 Stunden oder weniger, und bevorzugter in 4 Stunden oder weniger, oder in 5 Minuten oder 12 Stunden, vorzugsweise in 10 Minuten oder 8 Stunden, und bevorzugter in 15 Minuten oder 4 Stunden nach Verabreichung des oralen Dünnfilms an einen Patienten auflöst.

Es wird auch bevorzugt, dass sich die Rückschicht in Wasser, in künstlichem oder natürlichem Speichel oder in jedem anderen wässrigen Medium, vorzugsweise in einem gepufferten wässrigen Medium, bei 37 °C und 150 U/min, in 5 min oder mehr, vorzugsweise in 10 Minuten oder mehr, vorzugsweise in 15 Minuten oder mehr, und bevorzugter in 30 Minuten oder mehr, und in 15 Stunden oder weniger, vorzugsweise in 12 Stunden oder weniger, und bevorzugter in 10 Stunden oder weniger, oder in 10 Minuten oder 15 Stunden, vorzugsweise in 15 Minuten oder 12 Stunden, und bevorzugter in 30 Minuten oder 10 Stunden löst. Die Rückschicht kann hinsichtlich der Größe insbesondere gleich groß oder größer sein als die Matrixschicht. So sind in bestimmten Ausführungsformen die Größe der Rückschicht und die Größe der Matrixschicht gleich groß, während in anderen Ausführungsformen die Rückschicht größer ist als die Oberfläche der Matrixschicht und sich vergrößert. Während eine Schichtstruktur mit gleicher Größe von Rück- und Matrixschicht einfacher herzustellen ist, da ein zweilagiges Blatt gestanzt werden kann, um die Schichtstruktur bereitzustellen, ist eine Schichtstruktur mit einer Rückschicht, die größer als die Matrixschicht ist, schwieriger herzustellen, bietet aber auch den Vorteil, dass die Gefahr des aktiven Auslaufens geringer ist, da auch der Rand der Matrixschicht von der Trägerschicht bedeckt wird.

Die vorliegende Erfindung betrifft ferner ein Verfahren zur Herstellung des erfindungsgemäßen oralen Dünnfilms. Das Verfahren umfasst die Schritte:
a) Bereitstellen mindestens einer wirkstoffhaltigen Matrixschicht umfassend die Schritte
   a1) Herstellen einer Lösung oder Suspension umfassend das mindestens eine Polymer und den mindestens einen pharmazeutisch aktiven Wirkstoff und
   a2) Ausstreichen und Trocknen der gemäß Schritt a1) erhaltenen Lösung oder Suspension;
b) Bereitstellen mindestens einer Rückschicht mit einer Schichtdicke von 10 µm bis 500 µm, umfassend die Schritte
   b1) Herstellen einer Lösung oder Suspension umfassend mindestens ein Polymer, das freie Carboxylgruppen umfasst, wobei 10 bis 100% der freien Carboxylgruppen des mindestens einen Polymers, das freie Carboxylgruppen umfasst, neutralisiert als Salz vorliegen; wobei das mindestens eine Polymer, das freie Carboxylgruppen umfasst, in einer Menge von 10 bis 99 Gew.-%, bezogen auf das Gesamtgewicht der Rückschicht, in der Rückschicht vorhanden ist;
   b2) Ausstreichen und Trocknen der gemäß Schritt b1) erhaltenen Lösung; c) Zusammenfügen der gemäß a) erhaltenen wirkstoffhaltigen Matrixschicht und der gemäß b) erhaltenen Rückschicht, um einen mehrschichtigen oralen Dünnfilm zu erhalten.

Das Polymer, das in Schritt b) eingesetzt wird, kann durch Zugabe von mindestens einer Base, wie vorstehend beschrieben mindestens teilweise neutralisiert werden. Alternativ kann ein vom Hersteller bereits vorneutralisiertes Polymer eingesetzt werden.

Das Zusammenfügen der beiden Filme kann durch dem Fachmann geläufige Methoden erfolgen. So kann beispielsweise auf einen ersten Film mittels Beschichtung ein weiterer Film aufgebracht werden, wobei es unerheblich ist welcher Film auf welchen Film beschichtet wird, solange die Rückschicht eine der äußersten Schichten bildet. Ferner können die beiden Schichten durch eine Klebeschicht miteinander verbunden werden. Außerdem können die beiden Schichten mittels Wärmeeinwirkung miteinander verbunden werden.

Die vorliegende Erfindung betrifft ferner einen mehrschichtigen oralen Dünnfilm erhältlich nach dem vorstehend beschriebenen Verfahren.

Außerdem betrifft die vorliegende Erfindung einen mehrschichtigen oralen Dünnfilm, wie vorstehend beschrieben oder erhältlich nach dem vorstehend beschriebenen Verfahren, zur Verwendung als Arzneimittel.

Die vorliegende Erfindung betrifft zudem einen mehrschichtigen oralen Dünnfilm, wie vorstehend beschrieben oder erhältlich nach dem vorstehend beschriebenen Verfahren, als Arzneimittel zur Verwendung in der Behandlung von Schmerz und/oder Depressionen, insbesondere zur Reduzierung des Suizidrisikos und/oder zur Verwendung als Allgemeinanästhetikum, vorzugsweise zur Einleitung und Durchführung einer Vollnarkose oder als Ergänzung bei Regionalanästhesien und/oder als Analgetikum.

Die vorstehend für den erfindungsgemäßen mehrschichtigen oralen Dünnfilm aufgeführten bevorzugten Ausführungsformen gelten auch für das erfindungsgemäße Verfahren, den durch dieses Verfahren erhaltenen mehrschichtigen oralen Dünnfilm und für dessen Verwendung als Arzneimittel.

Beschreibung der Figuren:

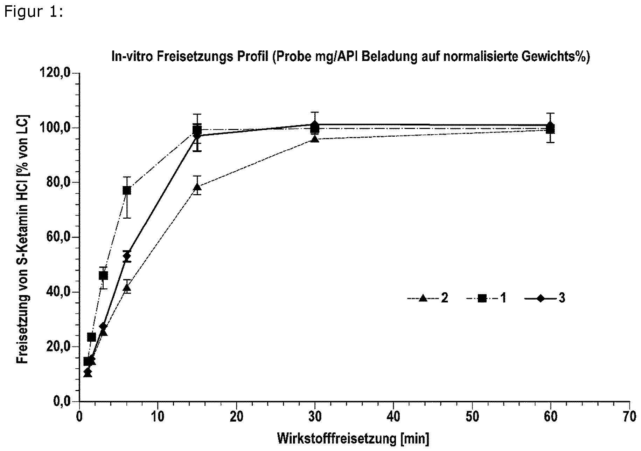

Figur 1: Auflösungszeiten von Formulierungen ohne Rückschicht, mit PET-Rückschicht und einer erfindungsgemäßen Rückschicht.

Die Erfindung wird nachfolgend anhand von nicht beschränkenden Beispielen näher erläutert.

### Beispiele:

### Beispiel 1:

Eine Rückschicht wird wie folgt hergestellt:
Formulierung Zusammensetzung:

**Tabelle 1:**

| **Inhaltsstoff** | **Funktion** | **Anteil [%]** |
|---|---|---|
| Kollicoat MAE 100-55 | Polymer | 42 |
| Kollocoat MAE 100 P | Polymer | 42 |
| Triethylcitrat | Weichmacher | 10 |
| Sucralose | Geschmackskorrigenz | 3 |
| Saccharin Na | Geschmackskorrigenz | 2 |
| Cherry Flavour | Geschmackskorrigenz | 1 |

Lösemittel: Ethanol:Wasser 80:20
Angestrebtes Flächengewicht: ca. 50 g/m2
Herstellabfolge:
Die Lösemittel werden zusammen vorgelegt und der Weichmacher und das Aroma wird zugegeben. Anschließend werden Süßungsmittel nacheinander gelöst und die Polymeren werden eingestreut und gelöst. Die Masse wird nun ruhen gelassen bis sie blasenfrei ist.

Es resultiert eine homogene leicht trübe Masse.

### Beschichtung/Trocknung:

Die Masse wird auf einen silikonisierten Liner aufgebracht und der daraus resultierende Film wird getrocknet.

Es resultiert ein dünner, stabiler, klarer Film.

### Weiterverarbeitung:

Der so erhaltene Strich kann nun weiterverarbeitet werden. So kann auf diese Rückschicht mittels Beschichtung, Verklebung oder Wärmeeinwirkung ein weiterer Film, z.B. die Matrixschicht, aufgebracht werden.

### Beispiel 2:

In einer ersten Untersuchung wurden drei Formulierungsvarianten getestet. Bei der Formulierung 1 handelte es sich um die Basis ohne Rückschicht. Aus der gleichen Masse wurde dann eine Formulierung mit PET Backing (unlöslich, 2) und der erfindungsgemäßen auflösenden Rückschicht (3) gebildet.

**Tabelle 2:**

| **Material** | **1** | **2** | **3** |
|---|---|---|---|
| **Matrixschicht** | | | |
| S-Ketamin HCl | 30.0 % | 30.0 % | 30.0 % |
| Kollidon VA 64¹ | 20.5 % | 20.5 % | 20.5 % |
| Kollicoat MAE² | 35.0 % | 35.0 % | 35.0 % |
| NaOH | 1.0 % | 1.0 % | 1.0 % |
| accharin Na | 2.0 % | 2.0 % | 2.0 % |
| Sucralose | 1.0 % | 1.0 % | 1.0 % |
| Cherry Flavor | 3.0 % | 3.0 % | 3.0 % |
| Glycerin | 7.5 % | 7.5 % | 7.5 % |
| Lösungsmittel | Aqua purificata / Ethanol | Aqua purificata / Ethanol | Aqua purificata / Ethanol |
| **Rückschicht** | - | | - |
| PET Rückschicht | - | FO PET 15µm tsp. | |
| Kollicoat MAE Rückschicht | - | - | Kollicoat MAE Rückschicht: |
| Kollicoat MAE 100-55 | - | - | 42.0 % |
| Kollicoat MAE 100 P | - | - | 42.0 % |
| Triethylcitrate | - | - | 10.0 % |
| Cherry EU | - | - | 3.0 % |
| Saccarin Na | - | - | 2.0 % |
| Sucralose | - | - | 1.0 % |
| Lösungsmittel | - | - | Aqua purificata / Ethanol |

| | | | |
|---|---|---|---|
| ¹: Vinylpyrrolidon/Vinylacetat-Copolymer ²: (Meth)Acrylsäure/Ethylacrylat-Copolymer | | | |

**Tabelle 3:**

| **Formulierung** | **Zerfallszeit [s]** | **Voll aufgelöst* [s]** |
|---|---|---|
| 1 | 341 | 425 |
| 2 | 773 | 1122 |
| 3 | Nicht erkennbar | 742 |

| | | |
|---|---|---|
| * Die volle Auflösezeit bezieht sich auf die Auflösung der mindestens einen pharmazeutisch aktiven Wirkstoff enthaltende Matrixschicht. | | |

Geprüft wurden die Zerfallsgeschwindigkeit und die Wirkstofffreisetzung.

Die Prüfung der Zerfallszeit erfolgte mittels eines Standard Zerfalls Tester mit Sinker und ist hinreichend bekannt und in den Arzneibüchern beschrieben. Die Wirkstofffreisetzung erfolgte mittels Standard Dissolution Tester mittels "Rotating Zylinder" Methode. Hierzu wurden die OTFs auf deinen Zylinder aufgeklebt der in dem Freisetzungsmedium rotierte. Die Analyse erfolgte über HPLC.

In den Versuchen konnte bestätigt werden, dass sich die Rückschicht der erfindungsgemäßen Formulierung 3 auflöste und den Zerfall und die Wirkstofffreisetzung gegenüber der Referenz (1) signifikant verlangsamte. Die Ergebnisse sind ferner in Figur 1 dargestellt.

Die Neutralisierung des Polymers erfolgt, in dem zu einem in Wasser suspendierten oder in einer Mischung aus organischem Lösemittel und Wasser gelösten Polymer (hier Kollicoat L100-55). Natronlauge (vorzugweise als Lösung in dem entsprechenden Lösemittel, welches auch für die Polymerlösung verwendet wurde) unter Rühren zugetropft wurde. Durch die zunehmende Neutralisation wird die Löslichkeit in dem organischen Lösemittel heruntergesetzt und die in Wasser erhöht.

Hierbei ist es bevorzugt, dass eine gewisse Menge an Wasser vorhanden ist, da sonst eine Phasentrennung stattfindet und das Polymer als fester Bestandteil ausfallen kann.

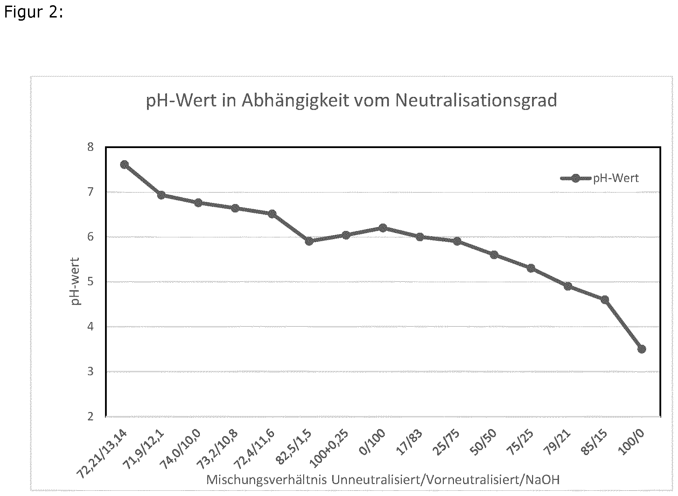

Da bei höheren Konzentration der Anteil an zugegebener Natronlauge sehr hoch wird wurde auch mit einem Blend an vorneutralisiertem Polymer (Kollicoat MAE 100P vorneutralisiert vom Hersteller mittels NaOH) und nicht neutralisiertem Polymer (Kollicoat MAE 100-55) gearbeitet (siehe Tabelle 4). Der pH Wert der in Tabelle 4 zusammengefassten Polymermischungen ist in Figur 2 dargestellt.

**Tabelle 4**

| Rezeptur | Kollicoat MAE 100-55 | Kollicoat MAE 100P | Verhältnis | NaOH [%] | pH-Wert |
|---|---|---|---|---|---|
| 1 | | 72.21 | 72.21/13.14 | 13,14 | 7,61 |
| 2 | | 71.9 | 71.9/12.1 | 12,1 | 6,93 |
| 3 | | 74 | 74.0/10.0 | 10 | 6,76 |
| 4 | | 73.2 | 73.2/10.8 | 10,8 | 6,64 |
| 5 | | 72.4 | 72.4/11,6 | 11,6 | 6,51 |
| 6 | | 82.5 | 82.5/1.5 | 1,5 | 5,9 |
| 7 | | 100 | 100+0.25 | 0,25 | 6,04 |
| 8 | 0 | 100 | 0/100 | 0 | 6,2 |
| 9 | 17 | 83 | 17/83 | 0 | 6 |
| 10 | 25 | 75 | 25/75 | 0 | 5,9 |
| 11 | 50 | 50 | 50/50 | 0 | 5,6 |
| 12 | 75 | 25 | 75/25 | 0 | 5,3 |
| 13 | 79 | 21 | 79/21 | 0 | 4,9 |
| 14 | 85 | 15 | 85/15 | 0 | 4,6 |
| 15 | 100 | 0 | 100/0 | 0 | 3,5 |

### Beispiel 3:

In einem weiteren Versuch wurden die Unterschiede zwischen verschiedenen pH-Werten dargestellt. Hierzu wurde ein Wirkstofffilm mit ähnlicher Zusammensetzung wie bei vorherigen Versuchen mit Rückschichten mit unterschiedlichen Neutralisationsgraden (pH-Werten) versehen. Die Zusammensetzungen (in Gew.-%) sind in Tabelle 5 zusammengefasst.

**Table 5:**

| **Formulierung** | **1** | **2** | **3** | **4** |
|---|---|---|---|---|
| **Matrixschicht** | | | | |
| S-Ketamine HCl | 30.0 % | 30.0 % | 30.0 % | 30.0 % |
| Kollidon VA 64 | 20.5 % | 20.5 % | 20.5 % | 20.5 % |
| Kollicoat MAE | 35.0 % | 35.0 % | 35.0 % | 35.0 % |
| NaOH | 1.0 % | 1.0 % | 1.0 % | 1.0 % |
| Saccharin Na | 2.0 % | 2.0 % | 2.0 % | 2.0 % |
| Sucralose | 1.0 % | 1.0 % | 1.0 % | 1.0 % |
| Cherry Flavor | 3.0 % | 3.0 % | 3.0 % | 3.0 % |
| Glycerin | 7.5 % | 7.5 % | 7.5 % | 7.5 % |
| Lösungsmittel | Aqua purificata / Ethanol | Aqua purificata / Ethanol | Aqua purificata / Ethanol | Aqua purificata / Ethanol |

| **Kollicoat MAE Rückschicht** | **Kollicoat MAE pH 3.5:** | Kollicoat **MAE pH 5.0:** | **Kollicoat MAE pH 6.0:** | **Kollicoat MAE pH 7.0:** |
|---|---|---|---|---|
| Kollicoat MAE 100-55 | 84.0 % | 66.4 % | 14.3 % | -- |
| Kollicoat MAE 100 P | -- | 17.6 % | 69.7 % | 71.9 % |
| Triethyl citrate | 10.0 % | 10.0 % | 10.0 % | 10.0 % |
| Cherry EU | 3.0 % | 3.0 % | 3.0 % | 3.0 % |
| Saccarin Na | 2.0 % | 2.0 % | 2.0 % | 2.0 % |
| Sucralose | 1.0 % | 1.0 % | 1.0 % | 1.0 % |
| NaOH | -- | -- | -- | 12.1 % |
| **Lösungsmittel** | Aqua purificata / Ethanol | Aqua purificata / Ethanol | Aqua purificata / Ethanol | Aqua purificata / Ethanol |

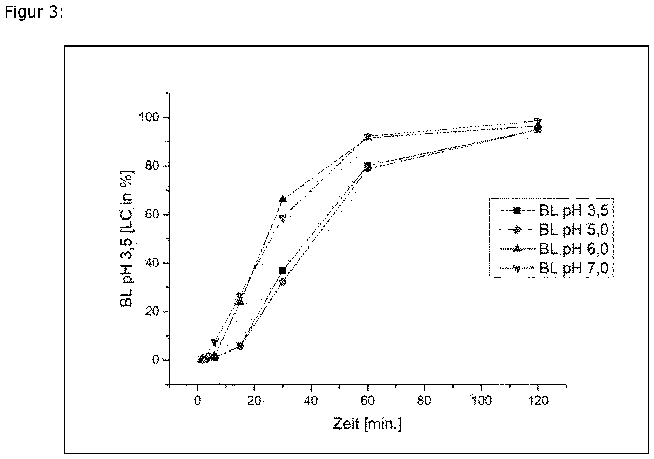

Die Freisetzung wurde in zwei Setups mit der "Paddle over disk" (TTS Halter) getestet. In einer erstes Variant wurde der OTF mit einem USP 5 TTS Halter Maschengröße 40 (35 mm Durchmesser) mit der API Seite zu einer PET Folie und der Rückschicht zum Freisetzungsmedium geprüft. Hierbei konnte die Wirkung der Rückschicht getestet werden. Die Messergebnisse sind in Figur 3 zusammengefasst.

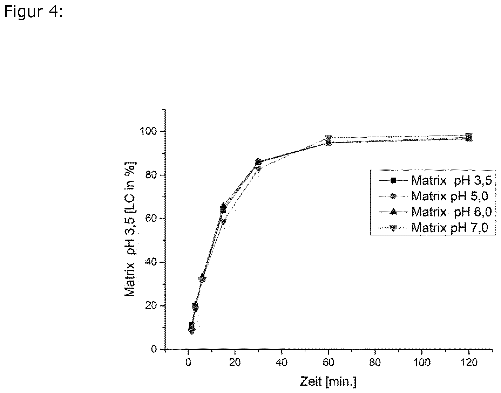

In einer zweiten Variante war das Setup im Wesentlichen gleich, mit dem Unterschied, dass die Rückschicht des OTFs zur PET Seite aufgebracht wurde um die Freisetzungsprofile der API Matrix zu zeigen. Die Messergebnisse sind in Figur 4 zusammengefasst.

### Beispiel 5:

Es wurden ferner orale Dünnfilme mit Agomelatin als pharmazeutisch aktivem Wirkstoff der in Tabelle 6 und 7 dargestellten Zusammensetzungen hergestellt.

**Tabelle 6:**

| **Agomelatin-haltige Matrixschicht** | | | | | |
|---|---|---|---|---|---|
| **Inhaltsstoff** | **Bsp. 3a** | | **Bsp. 3b, 3c und 3d** | | |
| | **Menge [g]** | **Feststoff [%]** | **Menge [g]** | | **Feststoff [%]** |
| Agomelatin | 0.50 | 9.96 | 3.50 | | 9.98 |
| Eucalyptol | 0.04 | 0.83 | 0.17 | | 0.49 |
| Menthol | 0.05 | 1.00 | 0.35 | | 1.00 |
| Methylsalicylat | 0.03 | 0.52 | 0.18 | | 0.51 |
| Novamint Fresh Peppermint | 0.18 | 3.52 | 1.24 | | 3.52 |
| Kolliphor RH 40 (Cremophor) | 0.12 | 2.43 | 0.72 | | 2.06 |
| FD&C Red #40 | 0.004 | 0.09 | 0.04 | | 0.10 |
| Sucralose | 0.03 | 0.50 | 0.18 | | 0.50 |
| Polyvinylpyrrolidon (Povidone K90) | 4.00 | 79.14 | 27.98 | | 79.80 |
| Polysorbat 80 (Tween 80) | 0.10 | 2.02 | 0.71 | | 2.03 |
| Ethanol | 21.89 | - | 155.60 | | - |
| Total | 26.94 | 100.01 | 190.67 | | 99.99 |
| Flächengewicht [g/m²] | 55.4 | | 50.0 | | |
| Agomelatingehalt [µg/cm²] | 551.6 | | 499.2 | | |
| Größe des OTF [cm²] | 0.522 | | | | |

| **Rückschicht** | | | | | |
|---|---|---|---|---|---|
| | **Ex. 3a** | **Ex. 3b** | **Ex. 3c** | | **Ex. 3d** |
| **Inhaltsstoff** | | | **Menge [g]** | **Festst. [%]** | FO-PET 15µm |
| Ethylcellulose N50F | | | 5.22 | 65.09 | |
| Rizinusöl | | | 2.80 | 34.91 | |
| Ethanol | | | 45.33 | - | |
| Total | | | 53.35 | 100.00 | |
| Flächengewicht [g/m²] | | | 12.3 | | |
| Größe des OTF [cm²] | 0.522 | | | | |

**Tabelle 7:**

| **Agomelatin-haltige Matrixschicht** | | | | | | | | |
|---|---|---|---|---|---|---|---|---|
| **Inhaltsstoff** | **Bsp. 3e - 3h** | | | | | | | |
| | **Menge [g]** | | | | **Feststoff [%]** | | | |
| Agomelatin | 3.50 | | | | 9.98 | | | |
| Eukalyptol | 0.17 | | | | 0.49 | | | |
| Menthol | 0.35 | | | | 1.00 | | | |
| Methylsalicylat | 0.18 | | | | 0.51 | | | |
| Novamint Fresh Peppermint | 1.24 | | | | 3.52 | | | |
| Kolliphor RH 40 (Cremophor) | 0.72 | | | | 2.06 | | | |
| FD&C Red #40 | 0.04 | | | | 0.10 | | | |
| Sucralose | 0.18 | | | | 0.50 | | | |
| Polyvinylpyrrolidone(Povidone K90) | 27.98 | | | | 79.80 | | | |
| Polysorbat 80 (Tween 80) | 0.71 | | | | 2.03 | | | |
| Ethanol | 155.60 | | | | | | | |
| Total | 190.67 | | | | 99.99 | | | |
| Flächengewicht [g/m²] | 50.0 | | | | | | | |
| Agomelatingehalt[µg/cm²] | 499.2 | | | | | | | |
| Größe des OTF [cm²] | 0.522 | | | | | | | |
| | | | | | | | | |

| **Rückschicht** | | | | | | | | |
|---|---|---|---|---|---|---|---|---|
| | **Bsp. 3e** | | **Bsp. 3f** | | **Bsp. 3g** | | **Bsp. 3h** | |
| **Inhaltsstoff** | **Menge [g]** | **Festst. [%]** | **Menge [g]** | **Festst. [%]** | **Menge [g]** | **Festst. [%]** | **Menge [g]** | **Festst. [%]** |
| Kollidon VA 64 | 9.29 | 46.36 | 9.31 | 39.63 | 8.40 | 42.11 | 8.50 | 42.50 |
| Eudragit L100-55 | 9.22 | 46.01 | 9.21 | 39.21 | 8.40 | 42.11 | 8.50 | 42.50 |
| Glycerol (99.5%) | 1.54 | 7.63 | 1.51 | 6.39 | 1.55 | 7.72 | 3.02 | 15.00 |
| NaOH 0.1N | - | - | 3.47 | 14.77 | 1.61 | 8.07 | - | - |
| Ethanol | 22.49 | - | 22.53 | - | 22.5 | - | 22.49 | - |
| Aqua purificata | 7.50 | - | 7.55 | - | 7.52 | - | 7.56 | - |
| Total | 50.04 | 100.00 | 53.58 | 100.00 | 49.98 | 100.01 | 50.07 | 100.00 |
| Flächengewicht [g/m²] | 26.8 | | 26.0 | | 20.5 | | 22.9 | |
| pH | 3.75 | | 5.01 | | 4.46 | | 3.62 | |
| Größe des OTF [cm²] | 0.522 | | | | | | | |

Für Beispiel 3a wurde ein Becherglas mit Agomelatin beladen. Ethanol, Eukalyptol, Menthol, Methylsalicylat, Novamint Fresh Peppermint, Kolliphor RH 40, FD&C Red #40, Sucralose und Polysorbat 80 wurden hinzugefügt und die Mischung dann gerührt. Das Polyvinylpyrrolidon wurde unter Rühren zugesetzt, und nach etwa 2,5 Stunden Rühren wurde eine klare, rote Lösung erhalten.

Für die Beispiele 3b bis 3h wurde die gleiche Beschichtungszusammensetzung für die agomelatinhaltige Schicht verwendet, die wie folgt hergestellt wurde: Ein Becherglas wurde mit Agomelatin gefüllt. Ethanol, Menthol, Eukalyptol, Methylsalicylat, Kolliphor RH 40, FD&C Red #40, Sucralose und Polysorbat 80 wurden zugegeben und die Mischung anschließend gerührt. Es wurde eine klare Lösung erhlten. Das Polyvinylpyrrolidon wurde zugegeben, und nach weiterem Rühren wurde das Novamint Fresh Peppermint unter Rühren tropfenweise zugegeben, um eine klare, rote Lösung zu erhalten.

### Herstellung einer zweiten Beschichtungszusammensetzung (Rückschicht):

Für Beispiel 3c wurde ein Becherglas mit Ethylcellulose beladen. Es wurde Ethanol zugegeben und die Mischung anschließend gerührt. Rizinusöl wurde unter Rühren zugegeben, um eine leicht opake Mischung zu erhalten.

Für die Beispiele 3e bis 3h wurde ein Becherglas mit Ethanol beladen. Aqua purificata und Kollidon wurden hinzugefügt und die Mischung wurde dann gerührt, um eine Lösung zu erhalten. Eudragit und Glycerin wurden unter Rühren zugegeben und es wurde eine klare Mischung erhalten.

Für die Beispiele 3f und 3g wurde Natriumhydroxidlösung zugegeben, um einen pH-Wert zu erhalten, wie in Tabelle 7 angegeben.

Die resultierende zweite Beschichtungszusammensetzung der Beispiele 3c und 3e bis 3h wurde auf eine Polyesterfolie (Polyethylenterephthalatfolie, einseitig silikonisiert, 75 µm dick, die als Trennschicht fungieren kann) aufgebracht und für etwa 10 min bei Raumtemperatur und 20 min bei 70 °C (Bsp. 3c) und für etwa 5 min bei Raumtemperatur, 10 min bei 35 °C und 2 min bei 80 °C (Bsp. 3e bis 3h) getrocknet. Die Schichtdicke ergab ein Flächengewicht von 12,3 g/m² (Bsp. 3c), 26,8 g/m² (Bsp. 3e), 26,0 g/m² (Bsp. 3f), 20,5 g/m² (Bsp. 3g) bzw. 22,9 g/m² (Bsp. 3h).

Für Beispiel 3d wurde eine handelsübliche Polyethylenterephthalatfolie mit 15 µm Dicke als Trägerschicht verwendet.

Die resultierende agomelatinhaltige erste Beschichtungszusammensetzung der Beispiele 3a und 3b wurde auf eine Polyesterfolie (Polyethylenterephthalatfolie, einseitig silikonisiert, 75 µm dick, die als Trennschicht fungieren kann) aufgebracht und für etwa 15 min bei Raumtemperatur und 5 min bei 70 °C (Bsp. 3a) oder für etwa 5 min bei Raumtemperatur, 10 min bei 50 °C und 2 min bei 90 °C (Bsp. 3b) getrocknet. Für die Beispiele 3a und 3b ist der getrocknete Film die endgültige agomelatinhaltige Schichtstruktur.

Die resultierenden agomelatinhaltigen Erstbeschichtungszusammensetzungen der Beispiele 3c und 3e bis 3h wurden auf die getrocknete Trägerschicht aufgebracht und für etwa 5 min bei Raumtemperatur, 10 min bei 50 °C und 2 min bei 90 °C getrocknet (Bsp. 3c und 3e bis 3h).

Die Schichtdicke ergab ein Flächengewicht von 55,4 g/m² (Bsp. 3a) bzw. 50,0 g/m² (3b, 3c und 3e bis 3h). Der Beschichtungsprozess von 3d war identisch mit dem der Beispiele 3b, 3c und 3e bis 3h, mit der Ausnahme, dass die Beschichtungszusammensetzung auf einen Polyethylenterephthalatfilm von 15 m Dicke aufgebracht wurde, wodurch ein OTF mit einer Trägerschicht (aus einem Polyethylenterephthalatfilm) entsteht.

Die OTFs der Beispiele 3b, 3c und 3e bis 3h wurden durch Laminieren der Trägerschichten auf die agomelatinhaltigen Schichten hergestellt. Die Trennschicht wurde vor dem Laminieren entfernt.

### Messung der Schleimhautpermeationsrate:

Die Permeatmenge und die entsprechenden Schleimhautpermeationsraten der nach den Beispielen 3a bis 3h hergestellten OTFs wurden durch in-vitro-Experimente gemäß der OECD-Richtlinie (verabschiedet am 13. April 2004) mit Schweinemukosa (Schleimhaut-Ösophagus) bestimmt. Mit einem Dermatom wurde die Schleimhaut bis zu einer Dicke von 400 µm mit einer intakten Barrierefunktion hergestellt. Die OTFs wurden mit einer Fläche von 0,522 cm² auf die Schleimhaut aufgetragen und die Schleimhaut mit dem OTF auf der Oberseite in künstlichen Speichel getaucht (die Unterseite steht in Kontakt mit dem Rezeptormedium, die Oberseite ist auf eine Schleimhautfläche von 1,145 cm² aufgeteilt). Die agomelatinpermeierte Menge im Rezeptormedium (Phosphatpufferlösung pH 7,4) bei einer Temperatur von 37 ± 1°C wurde gemessen und die entsprechende Schleimhautpermeationsrate berechnet. Die Ergebnisse sind in den Tabellen 8 und 9 sowie in den Abbildungen 3a und 3b dargestellt. Die Standardabweichung (SD) wurde in diesem Beispiel nach der n-Methode berechnet.

**Tabelle 8:**

| **Zeit [h]** | **Bsp. 3a (n = 3)** | | **Bsp. 3b (n = 3)** | | **Bsp. 3c (n = 3)** | | **Bsp. 3d (n = 3)** | |
|---|---|---|---|---|---|---|---|---|
| | **Rate** | **SD** | **Rate** | **SD** | **Rate** | **SD** | **Rate** | **SD** |
| **0.5** | 23.19 | 5.57 | 55.30 | 46.92 | 17.07 | 6.03 | 5.44 | 3.18 |
| **1** | 91.36 | 7.68 | 131.57 | 84.76 | 63.94 | 8.91 | 29.20 | 12.09 |
| **2** | 105.02 | 3.93 | 115.97 | 51.31 | 75.98 | 11.34 | 45.16 | 9.80 |
| **4** | 64.60 | 0.46 | 45.71 | 15.35 | 59.38 | 0.99 | 37.46 | 2.22 |
| **6** | 34.53 | 1.21 | 32.42 | 6.60 | 37.75 | 1.01 | 35.10 | 3.86 |

**Tabelle 9:**

| **Zeit [h]** | **Bsp. 3e (n = 3)** | | **Bsp. 3f (n = 3)** | | **Bsp. 3g (n = 3)** | | **Bsp. 3h (n = 3)** | |
|---|---|---|---|---|---|---|---|---|
| | **Rate** | **SD** | **Rate** | **SD** | **Rate** | **SD** | **Rate** | **SD** |
| **0.5** | 24.26 | 1.05 | 12.66 | 7.08 | 11.06 | 3.42 | 17.28 | 7.68 |
| **1** | 61.51 | 2.48 | 41.72 | 12.24 | 35.91 | 4.34 | 47.51 | 11.58 |
| **2** | 63.17 | 1.45 | 50.58 | 6.03 | 44.57 | 3.58 | 53.13 | 7.70 |
| **4** | 58.15 | 2.65 | 52.93 | 3.42 | 46.19 | 2.17 | 50.98 | 4.52 |
| **6** | 43.26 | 3.14 | 45.01 | 0.75 | 37.46 | 2.54 | 45.56 | 2.35 |

### Verwendung von Agomelatin:

Die Verwendung von Agomelatin nach 6 Stunden wurde basierend auf der kumulativen Permeatmenge nach 6 Stunden und dem anfänglichen Agomelatingehalt berechnet. Die Ergebnisse sind in Tabelle 10 dargestellt.

**Tabelle 10:**

| **Verwendung von Agomelatin nach 6 Stunden [%]** | | | | | | | |
|---|---|---|---|---|---|---|---|
| Bsp. 3a (n = 3) | Bsp. 3b (n = 3) | Bsp. 3c (n = 3) | Bsp. 3d (n = 3) | Bsp. 3e (n = 3) | Bsp. 3f (n = 3) | Bsp. 3g (n = 3) | Bsp. 3h (n = 3) |
| 65.4 | 68.9 | 62.3 | 41.6 | 61.9 | 54.8 | 47.2 | 55.8 |

Die in-vitro-Experimente zeigen eine gute Schleimhautpermeationsrate und eine gute Verwendung des Wirkstoffs. Die Beispiele 3c bis 3h im Vergleich zu den Beispielen 3a und 3b zeigen, dass auch bei Verwendung einer Rückschicht eine geringere, aber dennoch gute Permeation erreicht werden kann.

## Patentansprüche

1. Mehrschichtiger oraler Dünnfilm, umfassend eine mindestens ein Polymer und mindestens einen pharmazeutisch aktiven Wirkstoff enthaltende Matrixschicht und mindestens eine Rückschicht, wobei die mindestens eine Rückschicht eine Schichtdicke von 10 µm bis 500 µm aufweist und mindestens ein Polymer, das freie Carboxylgruppen aufweist, wobei 10 bis 100% der freien Carboxylgruppen des mindestens einen Polymers, das freie Carboxylgruppen umfasst, neutralisiert als Salz vorliegen, umfasst und wobei das mindestens eine Polymer, das freie Carboxylgruppen umfasst, in einer Menge von 10 bis 99 Gew.-%, bezogen auf das Gesamtgewicht der Rückschicht, in der Rückschicht vorhanden ist.

2. Mehrschichtiger oraler Dünnfilm gemäß Anspruch 1, wobei die mindestens eine Rückschicht einen pH von 3,5 bis 7 aufweist.

3. Mehrschichtiger oraler Dünnfilm gemäß irgendeinem der vorhergehenden Ansprüche, wobei der Gehalt an freien Carboxylgruppen in dem Polymer, das freie Carboxylgruppen umfasst, bezogen auf die mittlere Polymermasse 10 bis 40 Gew.-% beträgt.

4. Mehrschichtiger oraler Dünnfilm gemäß irgendeinem der vorhergehenden Ansprüche, wobei das mindestens eine Polymer, das freie Carboxylgruppen umfasst, ein Polymer auf Basis von (Meth)Acrylsäure und/oder auf Basis eines Copolymers von (Meth)Acrylsäure und (Meth)Acrylsäureestern umfasst.

5. Mehrschichtiger oraler Dünnfilm gemäß gemäß irgendeinem der vorhergehenden Ansprüche, wobei das mindestens eine Polymer, das freie Carboxylgruppen umfasst, ein (Meth)Acrylsäure/Ethylacrylat-Copolymer umfasst.

6. Mehrschichtiger oraler Dünnfilm gemäß irgendeinem der vorhergehenden Ansprüche, wobei die freien Carboxylgruppen des mindestens einen Polymers, das freie Carboxylgruppen umfasst, durch Zugabe mindestens einer Base, vorzugsweise NaOH, neutralisiert wurden.

7. Mehrschichtiger oraler Dünnfilm gemäß irgendeinem der vorhergehenden Ansprüche, wobei die Rückschicht mindestens einen Weichmacher, vorzugsweise Triethylcitrat, umfasst.

8. Mehrschichtiger oraler Dünnfilm gemäß irgendeinem der vorhergehenden Ansprüche, wobei die Matrixschicht mindestens ein wasserlösliches Polymer umfasst.

9. Mehrschichtiger oraler Dünnfilm gemäß Anspruch 9, wobei das mindestens eine wasserlösliche Polymer ausgewählt ist aus der Gruppe bestehend aus Stärke und Stärkederivaten, Dextranen; Cellulosederivaten, wie Carboxymethylcellulose, Hydroxypropylcellulose, Hydroxyethylcellulose, Hydroxypropylmethylcellulose, Hydroxypropylethylcellulose, Natriumcarboxymethylcellulose, Ethyl- oder Propylcellulose; Polyacrylsäuren, Polyacrylaten, Polyvinylpyrrolidonen, Vinvlpyrrolidon/Vinylacetat-Copolymer, Polyvinylalkoholen, Polyethylenoxidpolymeren, Polyacrylamiden, Polyethylenglycolen, Gelatine, Collagen, Alginaten, Pektin, Pullulan, Traganth, Chitosan, Alginsäure, Arabinogalaktan, Galaktomannan, Agar, Agarose, Carrageen und natürlichen Gummen.

10. Mehrschichtiger oraler Dünnfilm gemäß irgendeinem der vorhergehenden Ansprüche, wobei der mindestens eine pharmazeutisch aktive Wirkstoff ausgewählt ist aus der Gruppe, bestehend aus den Wirkstoffklassen der Analgetika, Hormone, Hypnotika, Sedativa, Antiepileptika, Weckaminen, Psychoneurotropika, Neuro-Muskelblockern, Antispasmodika, Antihistaminika, Antiallergika, Cardiotonika, Antiarrhythmika, Diuretika, Hypotensiva, Vasopressoren, Antidepressiva, Antitussiva, Expectorantia, Thyroidhormonen, Sexualhormonen, Antidiabetika, Antitumor-Wirkstoffen, Antibiotika, Chemotherapeutika und Narcotika, wobei der mindestens eine pharmazeutisch aktive Wirkstoff vorzugsweise Ketamin, besonders bevorzugt (S)-Ketamin, umfasst.

11. Mehrschichtiger oraler Dünnfilm gemäß irgendeinem der vorhergehenden Ansprüche, wobei die Matrixschicht jeweils ferner mindestens einen Hilfsstoff ausgewählt aus der Gruppe, umfassend Farbstoffe, Aromastoffe, Süßstoffe, Weichmacher, geschmacksmaskierende Mittel, Emulgatoren, Enhancer, pH-Regulatoren, Feuchthaltemittel, Konservierungsmittel und/oder Antioxidationsmittel, umfasst und die Rückschicht ferner mindestens einen Hilfsstoff ausgewählt aus der Gruppe, umfassend Farbstoffe, Aromastoffe, Süßstoffe, geschmacksmaskierende Mittel, Emulgatoren, Enhancer, pH-Regulatoren, Feuchthaltemittel, Konservierungsmittel und/oder Antioxidationsmittel, umfasst.

12. Verfahren zur Herstellung eines mehrschichtigen oralen Dünnfilms gemäß irgendeinem der Ansprüche 1 bis 11, umfassend die Schritte:
a) Bereitstellen mindestens einer wirkstoffhaltigen Matrixschicht umfassend die Schritte
a1) Herstellen einer Lösung oder Suspension umfassend das mindestens eine Polymer und den mindestens einen pharmazeutisch aktiven Wirkstoff und
a2) Ausstreichen und Trocknen der gemäß Schritt a1) erhaltenen Lösung oder Suspension;
b) Bereitstellen mindestens einer Rückschicht mit einer Schichtdicke von 10 µm bis 500 µm, umfassend die Schritte
b1) Herstellen einer Lösung oder Suspension umfassend mindestens ein Polymer, das freie Carboxylgruppen umfasst, wobei 10 bis 100% der freien Carboxylgruppen des mindestens einen Polymers, das freie Carboxylgruppen umfasst, neutralisiert als Salz vorliegen, wobei das mindestens eine Polymer, das freie Carboxylgruppen umfasst, in einer Menge von 10 bis 99 Gew.-%, bezogen auf das Gesamtgewicht der Rückschicht, in der Rückschicht vorhanden ist;
b2) Ausstreichen und Trocknen der gemäß Schritt b1) erhaltenen Lösung;
c) Zusammenfügen der gemäß a) erhaltenen wirkstoffhaltigen Matrixschicht und der gemäß b) erhaltenen Rückschicht, um einen mehrschichtigen oralen Dünnfilm zu erhalten.

13. Mehrschichtiger oraler Dünnfilm erhältlich durch das Verfahren gemäß Anspruch 12.

14. Mehrschichtiger oraler Dünnfilm gemäß irgendeinem der Ansprüche 1 bis 11 oder 13 zur Verwendung als Arzneimittel.

## Claims

1. A multi-layer oral thin film comprising a matrix layer, which contains at least one polymer and at least one pharmaceutically active agent, and at least one backing layer, wherein the at least one backing layer has a layer thickness of from 10 µm to 500 µm and comprises at least one polymer containing free carboxyl groups, wherein 10 to 100% of the free carboxyl groups of the at least one polymer comprising free carboxyl groups are present in a neutralised form as a salt, and wherein the at least one polymer comprising free carboxyl groups is provided in the backing layer in an amount of 10 to 99 wt.%, in relation to the total weight of the backing layer.

2. The multi-layer oral thin film according to claim 1, wherein the at least one backing layer has a pH of 3.5 to 7.

3. The multi-layer oral thin film according to any one of the preceding claims, wherein the content of free carboxyl groups in the polymer comprising free carboxyl groups is 10 to 40 wt.%, in relation to the mean polymer mass.

4. The multi-layer oral thin film according to any one of the preceding claims, wherein the at least one polymer comprising free carboxyl groups comprises a polymer based on (meth)acrylic acid and/or based on a copolymer of (meth)acrylic acid and (meth)acrylates.

5. The multi-layer oral thin film according to any one of the preceding claims, wherein the at least one polymer comprising free carboxyl groups comprises a (meth)acrylic acid/ethyl acrylate copolymer.

6. The multi-layer oral thin film according to any one of the preceding claims, wherein the free carboxyl groups of the at least one polymer comprising free carboxyl groups have been neutralised by addition of at least one base, preferably NaOH.

7. The multi-layer oral thin film according to any one of the preceding claims, wherein the backing layer comprises at least one plasticiser, preferably triethyl citrate.

8. The multi-layer oral thin film according to any one of the preceding claims, wherein the matrix layer comprises at least one water-soluble polymer.

9. The multi-layer oral thin film according to claim 9, wherein the at least one water-soluble polymer is selected from the group consisting of starch and starch derivatives, dextrans, cellulose derivatives, such as carboxymethyl cellulose, hydroxypropyl cellulose, hydroxyethyl cellulose, hydroxypropyl methylcellulose, hydroxypropyl ethyl cellulose, sodium carboxymethyl cellulose, ethyl or propyl cellulose, polyacrylic acids, polyacrylates, polyvinylpyrrolidones, vinyl pyrrolidone/vinyl acetate copolymer, polyvinyl alcohols, polyethylene oxide polymers, polyacrylamides, polyethylene glycols, gelatines, collagen, alginates, pectin, pullulan, tragacanth, chitosan, alginic acid, arabinogalactan, galactomannan, agar, agarose, carrageenan, and natural gums.

10. The multi-layer oral thin film according to any one of the preceding claims, wherein the at least one pharmaceutically active agent is selected from the group consisting of the active agent classes of analgesics, hormones, hypnotics, sedatives, antiepileptics, analeptics, psychoneurotropic drugs, neuro-muscle blockers, antispasmodics, antihistamines, antiallergics, cardiotonics, antiarrhythmics, diuretics, hypotensives, vasopressors, antidepressants, antitussives, expectorants, thyroid hormones, sexual hormones, antidiabetics, antitumour active agents, antibiotics, chemotherapeutics and narcotics, wherein the at least one pharmaceutically active agent is preferably ketamine, especially preferably (S) ketamine.

11. The multi-layer oral thin film according to any one of the preceding claims, wherein the matrix layer in each case also comprises at least one auxiliary substance selected from the group comprising colouring agents, flavourings, sweeteners, plasticisers, taste-masking agents, emulsifiers, enhancers, pH regulators, humectants, preservatives and/or antioxidants and the backing layer also comprises at least one auxiliary selected from the group comprising colouring agents, flavourings, sweeteners, taste-masking agents, emulsifiers, enhancers, pH regulators, humectants, preservatives and/or antioxidants.

12. A method for producing a multi-layer oral thin film according to any one of claims 1 to 11, comprising the steps of:
a) providing at least one active agent-containing matrix layer, comprising the steps of
a1) producing a suspension or suspension comprising the at least one polymer and the at least one pharmaceutically active agent, and
a2) spreading out and drying the solution or suspension obtained in accordance with step a1);
b) providing at least one backing layer with a layer thickness of from 10 µm to 500 µm, comprising the steps of
b1) producing a solution or suspension comprising at least one polymer comprising free carboxyl groups, wherein 10 to 100% of the free carboxyl groups of the at least one polymer comprising free carboxyl groups are present in a neutralised form as a salt, wherein the at least one polymer comprising free carboxyl groups is provided in the backing layer in an amount of 10 to 99 wt.%, in relation to the total weight of the backing layer;
b2) spreading out and drying the solution obtained in accordance with step b1);
c) joining together the active agent-containing matrix layer obtained in accordance with a) and the backing layer obtained in accordance with b) in order to obtain a multi-layer oral thin film.

13. A multi-layer oral thin film obtainable by the method according to claim 12.

14. A multi-layer oral thin film according to any one of claims 1 to 11 or 13 for the use as a medicament.

## Revendications

1. Film mince oral multicouche comprenant une couche de matrice contenant au moins un polymère et au moins un principe actif pharmaceutique et au moins une couche arrière, dans lequel l'au moins une couche arrière présente une épaisseur de couche de 10 µm à 500 µm et au moins un polymère qui présente des groupes carboxyles libres, dans lequel 10 à 100 % des groupes carboxyle libres de l'au moins un polymère qui comprend des groupes carboxyles libres sont présents de manière neutralisée sous forme de sel, et dans lequel l'au moins un polymère qui comprend des groupes carboxyles libres est présent dans la couche arrière en une quantité de 10 à 99 % en poids, par rapport au poids total de la couche arrière.

2. Film mince oral multicouche selon la revendication 1, dans lequel l'au moins une couche arrière présente un pH de 3,5 à 7.

3. Film mince oral multicouche selon l'une quelconque des revendications précédentes, dans lequel la teneur en groupes carboxyles libres dans le polymère qui comprend des groupes carboxyles libres est de 10 à 40 % en poids par rapport à la masse de polymère moyenne.

4. Film mince oral multicouche selon l'une quelconque des revendications précédentes, dans lequel l'au moins un polymère qui comprend des groupes carboxyles libres, comprend un polymère à base d'acide (méth)acrylique et/ou à base d'un copolymère d'acide (méth)acrylique et d'esters d'acide (méth)acrylique.

5. Film mince oral multicouche selon l'une quelconque des revendications précédentes, dans lequel l'au moins un polymère qui comprend des groupes carboxyles libres comprend un copolymère d'acide (méth)acrylique/acrylate d'éthyle.

6. Film mince oral multicouche selon l'une quelconque des revendications précédentes, dans lequel les groupes carboxyles libres de l'au moins un polymère qui comprend des groupes carboxyles libres ont été neutralisés par l'ajout d'au moins une base, de préférence du NaOH.

7. Film mince oral multicouche selon l'une quelconque des revendications précédentes, dans lequel la couche arrière comprend au moins un plastifiant, de préférence du citrate de triéthyle.

8. Film mince oral multicouche selon l'une quelconque des revendications précédentes, dans lequel la couche de matrice comprend au moins un polymère hydrosoluble.

9. Film mince oral multicouche selon la revendication 9, dans lequel l'au moins un polymère hydrosoluble est choisi dans le groupe composé d'amidon et dérivés d'amidon, dextranes ; dérivés de cellulose, tels que carboxyméthylcellulose, hydroxypropylcellulose, hydroxyéthylcellulose, hydroxypropylméthylcellulose, hydroxypropyléthylcellulose, carboxyméthylcellulose de sodium, éthylcellulose ou propylcellulose ; acides polyacryliques, polyacrylates, polyvinylpyrrolidones, copolymère de vinylpyrrolidone/acétate de vinyle, alcools polyvinyliques, polymères d'oxyde de polyéthylène, polyacrylamides, polyéthylène glycols, gélatine, collagène, alginates, pectine, pullulan, gomme adragante, chitosan, acide alginique, arabinogalactane, galactomannane, gélose, agarose, carraghénane et gommes naturelles.

10. Film mince oral multicouche selon l'une quelconque des revendications précédentes, dans lequel l'au moins un principe actif pharmaceutique est choisi dans le groupe composé des classes d'actifs des analgésiques, hormones, hypnotiques, sédatifs, antiépileptiques, amines réveillantes, psychoneurotropes, neurobloquants musculaires, antispasmodiques, antihistaminiques, antiallergiques, cardiotoniques, antiarythmiques, diurétiques, hypotenseurs, vasopresseurs, antidépresseurs, antitussifs, expectorants, hormones thyroïdiennes, hormones sexuelles, antidiabétiques, agents antitumoraux, antibiotiques, chimiothérapeutiques et narcotiques, dans lequel l'au moins un principe actif pharmaceutique comprend de préférence la kétamine, de manière particulièrement préférée la (S)-kétamine.

11. Film mince oral multicouche selon l'une quelconque des revendications précédentes, dans lequel la couche de matrice comprend en outre respectivement au moins un excipient choisi dans le groupe comprenant des colorants, arômes, édulcorants, plastifiants, masqueurs de goût, émulsifiants, exhausteurs, régulateurs de pH, agents humectants, conservateurs et/ou antioxydants, et la couche arrière comprend en outre au moins un excipient choisi dans le groupe, comprenant des colorants, arômes, édulcorants, masqueurs de goût, émulsifiants, exhausteurs, régulateurs de pH, agents humectants, conservateurs et/ou antioxydants.

12. Procédé de fabrication d'un film mince oral multicouche selon l'une quelconque des revendications 1 à 11, comprenant les étapes suivantes :
a) la mise à disposition d'au moins une couche de matrice contenant un principe actif comprenant les étapes suivantes
a1) la préparation d'une solution ou d'une suspension comprenant l'au moins un polymère et l'au moins un principe actif pharmaceutique et
a2) l'étalement et le séchage de la solution ou suspension obtenue conformément à l'étape a1) ;
b) la mise à disposition d'au moins une couche arrière d'une épaisseur de couche de 10 µm à 500 µm, comprenant les étapes suivantes
b1) la préparation d'une solution ou suspension comprenant au moins un polymère qui comprend des groupes carboxyles libres, dans lequel 10 à 100 % des groupes carboxyles libres de l'au moins un polymère qui comprend des groupes carboxyles libres, sont présents de manière neutralisée sous forme de sel, dans lequel l'au moins un polymère qui comprend des groupes carboxyles libres est présent dans la couche arrière en une quantité de 10 à 99 % en poids, par rapport au poids total de la couche arrière ;
b2) l'étalement et le séchage de la solution obtenue conformément à l'étape b1) ;
c) l'assemblage de la couche de matrice contenant le principe actif obtenue conformément à a) et de la couche arrière obtenue conformément à b) pour obtenir un film mince oral multicouche.

13. Film mince oral multicouche pouvant être obtenu par le procédé selon la revendication 12.

14. Film mince oral multicouche selon l'une quelconque des revendications 1 à 11 ou 13 pour une utilisation en tant que médicament.
